(19) Europäisches Patentamt / European Patent Office / Office européen des brevets

(11) **EP 2 489 300 B1**

(12) **EUROPEAN PATENT SPECIFICATION**

(45) Date of publication and mention
of the grant of the patent:
**22.07.2015 Bulletin 2015/30**

(51) Int Cl.:
*A61B 5/07* (2006.01)     *A61B 1/04* (2006.01)
*A61B 1/00* (2006.01)

(21) Application number: **10844749.1**

(22) Date of filing: **28.12.2010**

(86) International application number:
**PCT/JP2010/073793**

(87) International publication number:
**WO 2011/092995 (04.08.2011 Gazette 2011/31)**

(54) **METHOD FOR MANUFACTURING CAPSULE-TYPE MEDICAL DEVICE**

VERFAHREN ZUR HERSTELLUNG DER EINGEKAPSELTEN MEDIZINISCHEN VORRICHTUNG

PROCÉDÉ DE FABRICATION D'UN DISPOSITIF MÉDICAL DE TYPE CAPSULE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.01.2010 JP 2010019353**

(43) Date of publication of application:
**22.08.2012 Bulletin 2012/34**

(73) Proprietor: **OLYMPUS MEDICAL SYSTEMS CORP.
Tokyo 151-0072 (JP)**

(72) Inventor: **SEGAWA, Hidetake
Tokyo 151-0072 (JP)**

(74) Representative: **Schicker, Silvia
Wuesthoff & Wuesthoff
Patentanwälte PartG mbB
Schweigerstraße 2
81541 München (DE)**

(56) References cited:
WO-A1-2009/001666      JP-A- 5 023 322
JP-A- 2004 529 718     JP-A- 2007 516 765
JP-A- 2009 268 797     US-A1- 2007 106 112

**Description**

Field

The present invention relates to a method for manufacturing the capsule medical apparatus in corporating a plurality of function executing unit that execute predermined function in a capsule-shaped casing.

Background

A capsule medical apparatus which incorporates an imaging function, a wireless communication function, and the like in a capsule-shaped casing formed in a size allowing an insertion into an inside of a digestive canal of a subject such as a patient has appeared in the field of an endoscope. The capsule medical apparatus, after being swallowed from a mouth of the subject, travels an inside of a body, such as a digestive canal, of the subject according to its peristalsis and the like. The capsule medical apparatus sequentially captures images of the inside of the subject body and sequentially transmits the captured images wirelessly to a receiver placed outside of the subject until it is naturally excreted. Image data wirelessly transmitted from the capsule medical apparatus in this manner is imported into an image displaying device via the receiver and images are displayed statically or dynamically. A user such as a doctor observes the image data displayed on the image displaying device to make a diagnosis.

Besides, a capsule medical apparatus guidance system in which the capsule medical apparatus inserted into the inside of the subject body in the manner described above is guided by a magnetic force (magnetic guidance) has been proposed in recent years (see Patent Literature 1). A capsule medical apparatus to be magnetically guided generally includes in a casing a magnetic body such as a permanent magnet and a magnetic field generator placed outside of the subject applies a magnetic field to the capsule medical apparatus in the inside of the subject to magnetically guide the capsule medical apparatus to a desired position of the inside of the subject by the magnetic force of the applied magnetic field.

The capsule medical apparatus used in the capsule medical apparatus guidance system is preferably configured to have a desired range in density ($1.0 \pm 0.05$ [g/cm$^3$] and the like in density, for example) to perform a precise magnetic guidance by a magnetic field generated by a magnetic field generator. Here, the density is expressed by equation below and determined by a weight and a volume. The weight and the volume which determine the density are hereinbelow referred to as "physical parameters" collectively.

$$\mathtt{Density\ =\ Weight/Volume} \qquad \ldots(1)$$

Citation List

Patent Literature

Japanese Laid-open Patent Publication No. 2006-263167

However, since a capsule medical apparatus is constituted by a lot of parts, there has been a problem of individually causing a variation in physical parameters such as the weight and the volume due to a variation in dimension of the parts and a variation in assembly which is caused when the parts are assembled. As a result of this, the density of the capsule medical apparatus comes to have a variation, so that it is difficult to make the density fall within a desired range.

The present invention has been made in view of the above, and an object of the present invention is to provide a method for manufacturing the capsule medical apparatus capable of suppressing a variation in the density of the capsule medical apparatus.

US 2007/0106112 A1 discloses an in vivo device, system and a method for imaging a body lumen, typically liquid filled body lumen. The in vivo device may have a specific gravity of about 1 or a volume to weight ratio that enables it to float. The in vivo device may include a sensor, for example an image sensor, and may be attached to or set in one or more buoyant bodies. The in vivo device may be moved through the body lumen by the liquid movement in that lumen.

Summary

Solution to Problem

To solve the problems as described above and achieve the objet, a capsule medical apparatus not part of the present invention includes: a capsule-shaped casing; a plurality of function executing units that are housed in the casing to execute predetermined functions; and an adjuster that adjusts a value of a physical parameter for determining a density of the casing in which the plurality of function executing units are housed to a target value set in advance.

In the capsule medical apparatus not part of the present invention as set forth in the invention described above, the physical parameter is a weight of the casing in which the plurality of function executing units are housed, and the adjuster is a weight adjuster that adjusts a weight of the casing in which the plurality of function executing units are housed by performing increasing or decreasing a weight equivalent to a difference between the weight of the casing in which the plurality of function executing units are housed and the target value.

In the capsule medical apparatus not part of the present invention as set forth in the invention described above, the weight adjuster is a solid adjusting member which is a separate body from the function executing units and has a weight equivalent to the difference, and performs an adjustment by increasing, by being housed in the casing in which the plurality of function executing units are housed, the weight of the casing.

In the capsule medical apparatus not part of the present invention as set forth in the invention described above, the weight adjuster is a plurality of solid adjusting members which are separate bodies from the function executing unit and have a same weight, and performs an adjustment by increasing, when the solid adjusting members whose total weight becomes equivalent to the difference are housed in the casing in which the plurality of function executing units are housed, the weight of the casing.

In the capsule medical apparatus not part of the present invention as set forth in the invention described above, the weight adjuster is a paste form adjusting member which is a separate body from the function executing units and has a weight equivalent to the difference, and performs an adjustment by increasing, when being housed in the casing in which the plurality of function executing units, the weight of the casing.

In the capsule medical apparatus not part of the present invention as set forth in the invention described above, the weight adjuster is a part which serves as one or both of a part constituting the function executing units and a part housed in the casing for retaining the function executing units in the casing and has a weight equivalent to the difference, and performs an adjustment by increasing or decreasing the weight of the casing in which the plurality of function executing units are housed.

In the capsule medical apparatus not part of the present invention as set forth in the invention described above, the physical parameter is a volume of the casing in which the plurality of function executing units are housed, the casing is formed by a plurality of exterior members which are fitted in a predetermined fitting direction in assembly, and the adjuster is a volume adjuster that adjusts the volume of the casing in which the plurality of function executing units are housed to a target value by adjusting a length between ends in the fitting direction of the casing when the plurality of external members are fitted.

In the capsule medical apparatus not part of the present invention as set forth in the invention described above, the volume adjuster adjusts the length between the ends in the fitting direction of the casing by an inner pressure of the casing.

A method for manufacturing a capsule medical apparatus according to the present invention is defined in claim 1. measuring step of measuring a weight of a capsule-shaped

In the method for manufacturing a capsule medical apparatus according to the present invention as set forth in the invention described above, the adjusting step includes a selecting step of selecting a solid adjusting member which has a weight equivalent to the calculated difference among a plurality of solid adjusting members which are prepared in advance and each of which has a different weight, and the weight of the casing in which the plurality of function executing units are housed is increased by housing the selected solid adjusting member in the casing at the adjusting step.

In the method for manufacturing a capsule medical apparatus according to the present invention as set forth in the invention described above, the adjusting step includes a determining step of determining a number of the solid adjusting members which are prepared in advance and have a same weight so that a total weight of the solid adjusting members becomes equivalent to the calculated difference, and the weight of the casing in which the plurality of function executing units are housed is increased by housing the determined numbers of solid adjusting members in the casing at the adjusting step.

In the method for manufacturing a capsule medical apparatus according to the present invention as set forth in the invention described above, the weight of the casing in which the plurality of function executing units are housed is increased, at the adjusting step, by housing a paste form adjusting member which has a weight equivalent to the calculated difference.

In the method for manufacturing a capsule medical apparatus not part of the present invention as set forth in the invention described above, the adjusting step includes a selecting step of selecting a part which has a weight equivalent to the calculated difference among a plurality of parts which are prepared in advance so as to serve as one or both of a part constituting the function executing units and a part housed in the casing for retaining the function executing units in the casing and each of which has a different weight, and the weight of the casing in which the plurality of function executing units are housed is increased or decreased by housing the selected part in the casing at the adjusting step.

A method for manufacturing a capsule medical apparatus according to the present invention includes: an assembling step of assembling a capsule-shaped casing by arranging a plurality of function executing units that execute predetermined functions in an inside of a plurality of exterior members and fitting the plurality of exterior members in a predetermined fitting direction with the arrangement kept; a regulating step of regulating a length between ends in the fitting direction of the assembled casing to a length set in advance depending on a target volume of the casing in which the plurality of function executing units are housed; and a jointing step of jointing the exterior members with the length between the ends in the fitting direction of the casing regulated, wherein a jig which is provided with supporters that are fixed at an interval corresponding to the length set in advance and support the ends in the fitting direction of the casing is used to regulate the length between the ends in the fitting direction of the casing at the regulating step.

Advantageous Effects of Invention

The present invention is configured to be provided with the adjuster that adjusts a value of physical parameters which determine a density of a capsule medical apparatus to a target value set in advance, so that a variation in the density of the capsule medical apparatus can be suppressed by the adjuster.

Brief Description of Drawings

FIG. 1 is an explanatory view of a principle of adjusting a weight of a capsule medical apparatus according to a first embodiment.
FIG. 2 is a schematic explanatory view of the capsule medical apparatus and a weight adjusting member in a first specific example.
FIG. 3 is a schematic view of a state in which a weight adjusting member in a third specific example is added to the inside of the capsule medical apparatus.
FIG. 4 is a perspective view of a principal part of a side surface of a resin part according to a second embodiment.
FIG. 5 is a perspective view of a principal part of a side view of another resin part according to the second embodiment.
FIG. 6 is a perspective view of a principal part of a side surface of a resin part according to a modification.
FIG. 7 is a plane view of the resin part shown in FIG. 6.
FIG. 8 is a perspective view of a principal part of a side surface of a resin part according to another modification.
FIG. 9 is an explanatory view of a process of a method for manufacturing a capsule medical apparatus according to a third embodiment.
FIG. 10 is an explanatory view of a process subsequent to that shown in FIG. 9.
FIG. 11 is an explanatory view of a process subsequent to that shown in FIG. 10.
FIG. 12 is a side view of a jig according to a modification.
FIG. 13 is a perspective view of a container and a cap of a capsule medical apparatus according to a fourth embodiment.
FIG. 14 is an explanatory view of a fitting process and a regulating process according to the fourth embodiment.
FIG. 15 is a cross sectional view of a state in which the container and the cap are fitted according to the fourth embodiment.
FIG. 16 shows a groove part according to a modification.
FIG. 17 shows a groove part according to another modification.
FIG. 18 shows a groove part according to still another modification.

Description of Embodiments

Exemplary embodiments of a capsule medical apparatus according to the present disclosure will be explained in detail below with reference to the accompanying drawings. The present disclosure is not limited to the embodiments. In the description of the drawings, the same reference symbol is assigned to portions identical with each other.

(First embodiment)

First of all, a first embodiment will be explained. The first embodiment is configured to adjust a weight as one of the physical parameters which determine a density of a capsule medical apparatus to a target range set in advance and thereby suppress a variation in density of the capsule medical apparatus. Specifically, the capsule medical apparatus is manufactured by incorporating (adding) therein a weight adjusting member which serves as a weight adjuster as one example of an adjuster in the first embodiment. Here, the target range indicates a predetermined range whose median is the target value of a weight (target weight) of the capsule medical apparatus which needs adjusting, and a weight of the capsule medical apparatus is adjusted by tolerating an increase and a decrease by a predetermined amount with respect to the target weight in the first embodiment. A plurality of weight adjusting members each having a different weight are prepared in advance and the weight of the capsule medical apparatus is adjusted to fall within a desired target range by the weight of the weight adjusting member to be incorporated therein.

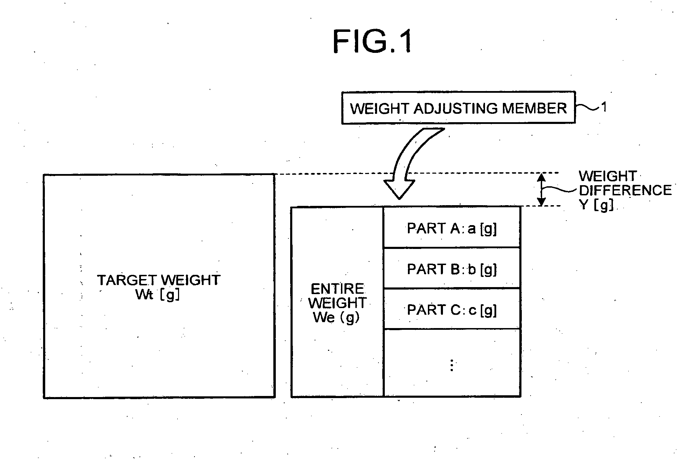

FIG. 1 is an explanatory view of a principle of adjusting a weight of a capsule medical apparatus according to the first embodiment. For example, in manufacturing a capsule medical apparatus, each weight of all parts (a part A, a part B, a part C, ...) constituting the capsule medical apparatus is measured prior to the manufacturing and a total weight (entire weight) is calculated (measuring process). Here, the target weight of the capsule medical apparatus is assumed to be Wt[g] as shown in FIG. 1. In contrast, the entire weight, calculated in the above-described manner, of all parts to be assembled is assumed to be We[g] and the entire weight We is assumed to be less than the target weight Wt by a weight difference Y. The weight difference Y is calculated by equation below (2)(difference calculating process).

$$\text{Weight difference } Y = \text{Entire weight } We - \text{Target weight } Wt \quad \ldots(2)$$

In this case, the weight of the capsule medical apparatus is increased and adjusted by adding a weight adjusting member 1 whose weight is equivalent to the weight difference Y (adjusting process) to manufacture the capsule medical apparatus. For example, the capsule medical apparatus is manufactured by arranging the weight adjusting member 1 at a position which is an inner space such as a clearance and the like among parts constituting the capsule medical apparatus. Here, a process of measuring the weight of the capsule medical apparatus may be performed after manufacturing the capsule medical apparatus.

This weight adjustment by adding the weight adjusting member 1 is performed for each capsule medical apparatus. In other words, the entire weight of all parts constituting each capsule medical apparatus is calculated for each capsule medical apparatus. Based on the weight difference Y between the target weight Wt and the calculated entire weight We, the weight adjusting member 1 whose weight is equivalent to the weight difference Y is selectively added to each capsule medical apparatus. This configuration allows revamping a variation in weight of an individual part due to a variation in dimension and the like and adjusting a weight of a capsule medical apparatus to be manufactured within a target range. It should be noted that a measure to set a stricter dimensional tolerance or to select parts based on the weight for suppressing a variation in weight of a capsule medical apparatus would lead to an increase in cost of parts.

As explained above, the capsule medical apparatus can be manufactured by selectively adding the weight adjusting member 1 whose weight is equivalent to the weight difference Y between the target weight Wt and the entire weight We of all parts constituting the capsule medical apparatus according to the first embodiment. This configuration allows suppressing a variation in density which is determined based on the weight of the capsule medical apparatus since the weight of each capsule medical apparatus to be manufactured can be made to fall within a desired target range.

<First illustrative embodiment>

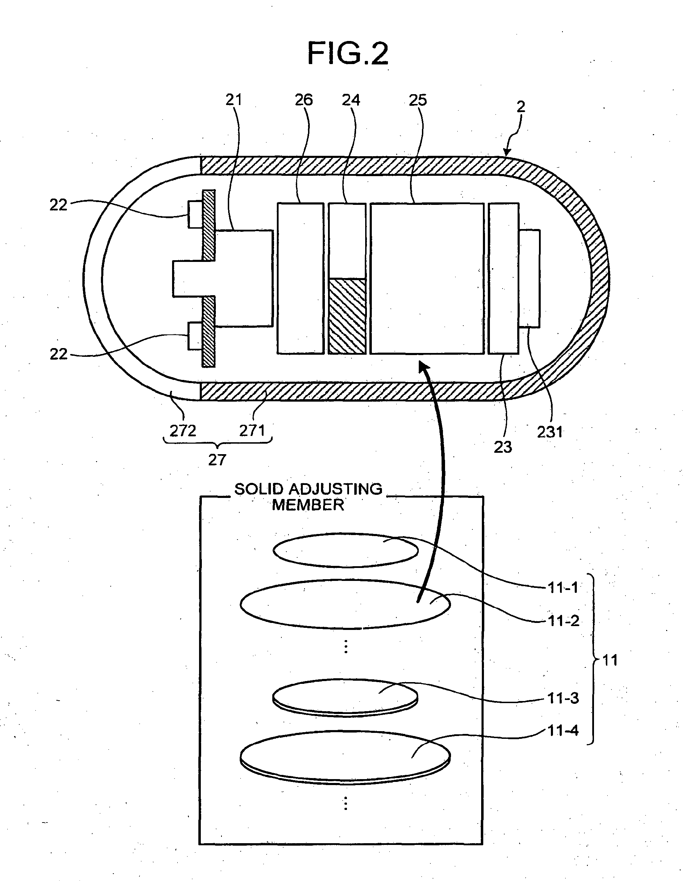

Next, a solid adjusting member which serves as the weight adjusting member in a first specific example will be explained. FIG. 2 is a schematic explanatory view of a capsule medical apparatus 2 and a solid adjusting member 11 (11-1, 11-2, 11-3, 11-4, ...) in the first specific example of the weight adjusting member to be selectively added to the inside of the capsule medical apparatus 2.

The capsule medical apparatus 2 is swallowed from a mouth of a subject and inserted to the inside of the subject to examine the inside of a lumen in the body such as a digestive canal, and incorporates therein a magnetic body such as a permanent magnet in addition to a plurality of function executing units which realize the imaging function and the wireless communication function. The capsule medical apparatus 2 is, after being inserted to the inside of the subject, magnetically guided by a magnetic field generated by a magnetic field generator placed outside of the subject. It is very important in the capsule medical apparatus 2 of such a type as to be magnetically guided in the inside of the lumen in the body in this manner to make the density of the capsule medical apparatus fall within a desired range for precisely performing the magnetic guidance.

Specifically, the capsule medical apparatus 2 is provided with an imaging unit 21 that captures images of the inside of the lumen in the body to obtain image data; an illumination unit 22 that irradiates the inside of the lumen in the body with a light; a wireless communication unit 23 that wirelessly transmits the image data obtained by the imaging unit 21 via an antenna 231; a permanent magnet 24 that enables a magnetic guidance by the magnetic field generator;

a power source unit 25 that supplies an electric power to the units constituting the capsule medical apparatus 2; a control unit 26 that controls operations of the units; and the like, and is configured such that each of those units is arranged at an appropriate position within a capsule casing 27 which has a capsule shape. More specifically, the imaging unit 21, the illumination unit 22, the wireless communication unit 23, the power source unit 25, the control unit 26, and the like are mounted on a not shown base plate and housed in the capsule casing 27.

The capsule casing 27 is formed in a size to be swallowed by a human. The capsule casing 27 includes a container 271 having nearly cylindrical shape whose one end has an opening and the other end has a dome-like shape (hemisphere shape) and a cap 272 having a dome-like shape, and the two exterior members are fitted to each other with the units housed inside in a longitudinal direction (fitting direction) of the capsule casing 27 to seal the inside of the container 271. The cap 272 is formed by a transparent member and serves as an optical window. Specifically, the imaging unit 21 and the illumination unit 22 are arranged in a manner of facing the cap 272 in the capsule casing 27, and the cap 272 transmits the illumination light from the illumination unit 22 to the outside of the capsule casing 27 and guides a reflection light to the inside of the capsule casing 27 at the same time.

The solid adjusting member 11 (11-1, 11-2, 11-3, 11-4, ...) which is housed in the capsule casing 27 in manufacturing the capsule medical apparatus 2 and added in the capsule medical apparatus 2 in the first specific example has a circular shape formed in a sheet state in a planar view. In the first specific example, the solid adjusting member 11 (11-1, 11-2, 11-3, 11-4, ...) of plural kinds each having a different weight is realized by preparing plural kinds each having a different diameter and thicknesses. Though a material of the solid adjusting member 11 is not specifically limited, plural kinds of members each having a different weight are prepared by adopting solid materials such as metal and plastic, and varying a shape, a size, and the like. A weight of the heaviest solid adjusting member 11 and a weight of the lightest solid adjusting member 11 can be set by assuming in advance a range of a variation in weight of the capsule medical apparatus based on a variation in dimension and the like of each of the parts constituting the capsule medical apparatus.

In manufacturing the capsule medical apparatus 2, an entire weight of the capsule medical apparatus 2 is first calculated in manufacturing the capsule medical apparatus 2. Here, the arrangement of the units including the imaging unit 21, the illumination unit 22, the wireless communication unit 23, the permanent magnet 24, the power source unit 25, the control unit 26, and the like in the inside of the capsule casing 27 is only schematically shown in FIG. 2, the shape and the arrangement location of parts constituting each unit are not presented accurately, and parts necessary other than the shown parts are arbitrarily arranged in the inside of the capsule casing 27. The entire weight of the capsule medical apparatus 2 is a total of the weight of the capsule casing 27 as an exterior member and the weight of all parts housed in the capsule casing 27. After calculating the entire weight, the solid adjusting member 11 whose weight is equivalent to the weight difference between the target weight and the calculated entire weight is then selected (selecting process). After that, while all parts are assembled and housed in the capsule casing 27 to manufacture the capsule medical apparatus 2 (housing process), the selected solid adjusting member 11 is arranged at a position which is an empty space inside the capsule casing 27 after the assembly and the like and housed in the capsule casing 27 on this occasion. Thus, the weight of the capsule medical apparatus 2 is increased by the weight of the housed solid adjusting member 11, so that the weight of the capsule medical apparatus 2 is adjusted within the target range.

According to the first specific example, the weight of the capsule medical apparatus 2 can be adjusted without detracting an assembly performance since the selected solid adjusting member 11 of a sheet state can be placed in an empty space in the inside of the capsule casing 27, for example in a clearance among parts directly, by folding, or the like thanks to the solid adjusting member 11 being a sheet state.

Here, the outer shape of the solid adjusting member 11 is not limited to the circular shape and may be a sheet state formed in a rectangular shape in a planar view such as a square and a rectangle. Besides, while the solid adjusting member 11 formed in a sheet state is shown in FIG. 2, the shape is not limited to the sheet state and any arbitrary shapes maybe adopted as long as the weight varies.

<Second illustrative embodiment>

Next, a solid adjusting member which serves as the weight adjusting member in a second specific example will be explained. In the specific example 1 described above, a plurality of solid adjusting members each having a different weight are prepared. In contrast, a lot of solid adjusting members having the same weight may be prepared. In the case of adjusting the weight of the capsule medical apparatus by using the solid adjusting member in the second specific example, the number of solid adjusting members whose total weight becomes equivalent to the weight difference is first determined based on the weight difference (determining process). After that, the determined numbers of solid adjusting members are arranged at an appropriate position such as an empty space inside the capsule casing and added in the capsule medical apparatus.

According to the second specific example, the solid adjusting member can be manufactured easily since it is only necessary to prepare a lot of solid adjusting members having the same weight. In addition, a lighter weight of one solid adjusting member enables a finer weight adjustment. Thus, the weight of the capsule medical apparatus can be adjusted as precisely close to the target weight as possible.

<Third illustrative embodiment>

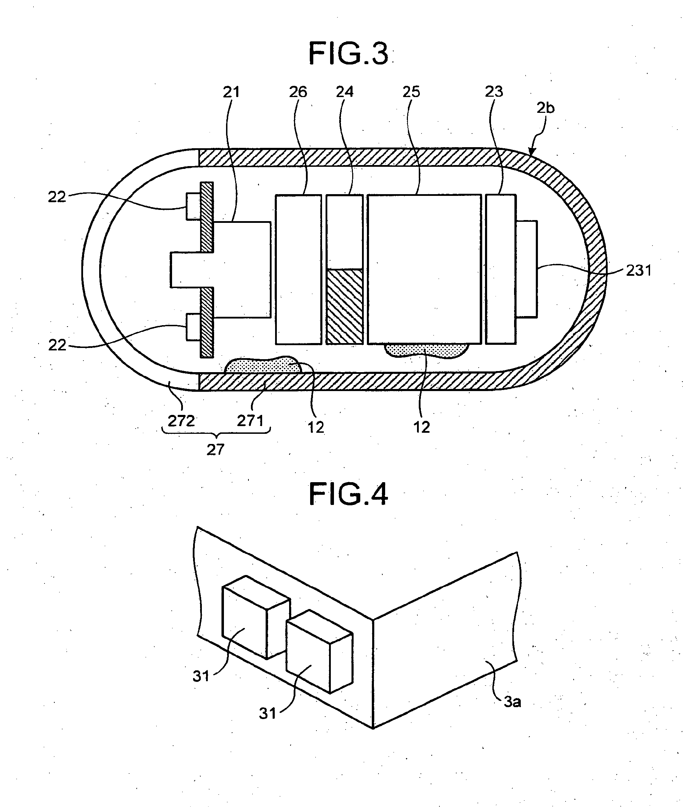

Next, a paste form adjusting member which serves as the weight adjusting member in a third specific example will be explained. FIG. 3 is a schematic view of a state in which a past form adjusting member 12 according to the third specific example is added in a capsule medical apparatus 2b. In FIG. 3, a constituent identical to the first specific example is assigned with the same reference symbol. In the third specific example, a paste form material such as clay is used as a material of the paste form adjusting member 12. The paste form adjusting member 12 whose weight is equivalent to the weight difference is arranged at a position as an empty space in the inside of the capsule casing 27 after the assembly, for example, at a position where the adjusting member does not interfere with other parts on a side surface or an upper surface of a part which is housed, by being mounted on a not-shown base plate and the like, in the inside of the capsule casing 27, or on an inner wall surface and the like of the capsule casing 27. By arranging the paste form adjusting member 12 in this manner, the capsule medical apparatus 2b is manufactured with the addition of the paste form adjusting member 12 whose weight is equivalent to the weight difference. Thus, the weight of the capsule medical apparatus 2b is adjusted within the target range. The paste form adjusting member 12 may be arranged in some portions at appropriate positions inside the capsule casing 27 so that the total of the weight of the paste form adjusting member 12 becomes nearly equal to the weight difference.

The weight adjustment of the capsule medical apparatus with the fixed adjusting member using a material whose weight is fixed like the first and the second specific examples described above as the weight adjusting member is a step-by-step approach. In contrast to this, the paste form adjusting member 12 using a paste material such as clay like the third specific example enables nearly single step adjustment since the weight of the paste form adjusting member 12 can be arbitrarily adjusted. Thus, the weight of the paste form adjusting member 12 can be made almost as much amount as the weight difference, so that the weight of the capsule medical apparatus 2b can be made nearly equivalent to the target weight.

The paste form material is not limited to clay and an adhesive agent may be, for example, used instead of clay. In the case of using an adhesive agent as a material of the paste form adjusting member 12, a dispenser can be used to perform a fine control of a discharging amount and thereby a weight adjustment of the capsule medical apparatus 2b can be performed with higher precision. Besides, the paste form adjusting member 12 added by using a dispenser in this manner in the capsule medical apparatus 2b is hardened via a heating treatment, an exposure to ultraviolet radiation, or the like. Thus, a situation in which the paste form adjusting member 12 moves in the capsule casing 27 due to a vibration and the like can be prevented in using the capsule medical apparatus 2b.

(Second embodiment)

A second embodiment will be explained next. The second embodiment is configured, similarly to the first embodiment, to adjust the weight of a capsule medical apparatus within a target range and thereby suppress a variation in density of the capsule medical apparatus. In the first embodiment described above, the weight adjusting member is prepared in a separate body from parts constituting the capsule medical apparatus. In contrast, the weight of the capsule medical apparatus to be manufactured is adjusted by a weight of parts constituting the capsule medical apparatus in the second embodiment.

For example, there is a case of using a resin part for retaining the base plate on which the imaging unit, the illumination unit, the wireless communication unit, the control unit, and the like are mounted and which is housed in the capsule casing and fixing this base plate in the inside of the capsule casing in manufacturing the capsule medical apparatus. In the second embodiment, resin parts of plural kinds each having a different weight are prepared in advance by varying the shape of the resin part. Then, based on the weight different between the target weight and the entire weight of the capsule medical apparatus, a resin part having a weight which allows the entire weight to fall within the target weight is selected and used among the prepared resin parts of plural kinds to manufacture the capsule medical apparatus.

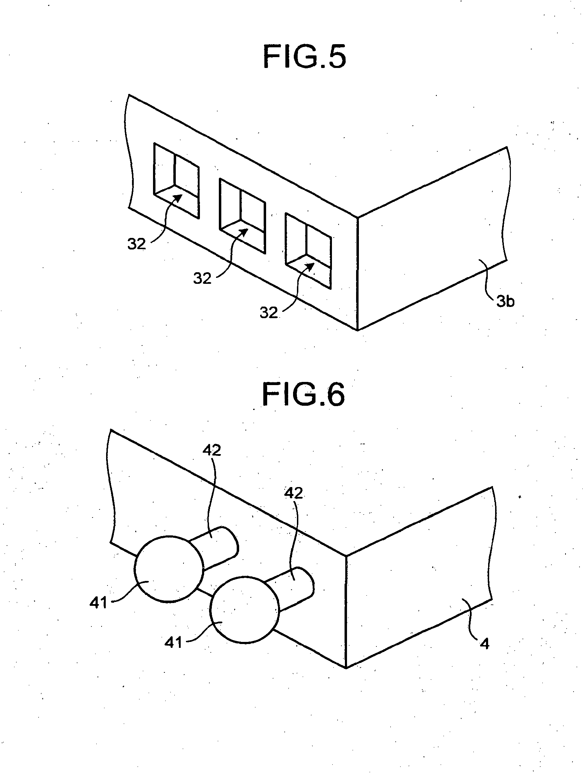

FIGS. 4 and 5 are explanatory views of a specific weight adjusting method using resin parts each having a different shape and perspective views of principal parts of the resin parts 3a and 3b, respectively. The resin part 3a shown in FIG. 4 is provided with two protrusions 31 on its side surface. In contrast to the resin part 3a, the resin part 3b shown in FIG. 5 is provided with three holes 32 on its side surface.

In the second embodiment, a plurality of resin parts provided with the protrusion 31 whose number is different from each other are prepared as a resin part provided with the protrusions 31 like the resin part 3a shown in FIG. 4. Similarly, a plurality of resin parts provided with the hole 32 whose number is different from each other are prepared as a resin part provided with the holes 32 like the resin part 3b shown in FIG. 5. By preparing resin parts provided with the protrusion 31 or the hole 32 whose number is different from each other, resin parts of plural kinds each having a different weight (the resin parts 3a and 3b, for example) are realized.

The resin parts of plural kinds each having a different weight may be prepared by varying not only the number of the protrusions 31 but also the height, the shape, the cross-section area, and the like of the protrusion 31, for example. Similarly, the resin parts of plural kinds each having a different weight may be prepared by varying not only the number of the holes 32 but also the depth, the shape, the opening area, and the like of the holes 32, for example. While the protrusion 31 and the hole 32 are shown by being formed on the side surfaces of the resin parts 3a and 3b in FIGS. 4 and 5, respectively, the position where the protrusion 31 and the hole 32 are formed is not specifically limited. Especially, the protrusion 31 is formed at a position which does not cause an interference with other parts when parts are assembled.

As explained above, the weight of the capsule medical apparatus can be adjusted within the target range by using a resin part which is a part constituting the capsule medical apparatus according to the second embodiment. In the weight adjustment using the weight adjusting member 1 according to the first embodiment, the weight adjustment cannot be performed when the entire weight of the capsule medical apparatus is heavier than the target weight. In contrast to this, since parts of plural kinds each having a different weight (the resin parts 3a and 3b, for example) are prepared as a resin part constituting the capsule medical apparatus in the second embodiment, when the entire weight of the capsule medical apparatus is heavier than the target weight, a resin part whose weight is lighter by the difference in weight is selected and used to decrease the entire weight, so that the entire weight can be adjusted within the target range. On the other hand, when the entire weight of the capsule medical apparatus is lighter than the target weight, a resin part whose weight is heavier by the difference in weight is selected and used to increase the entire weight, so that the entire weight can be adjusted within the target range. In this manner, a weight adjustment can be performed even when the entire weight of the capsule medical apparatus is heavier than the target weight according to the second embodiment.

While the resin part provided with the protrusion 31 (the resin part 3a, for example) and the resin part provided with the hole 32 (the resin part 3b, for example) are prepared as a resin part constituting the capsule medical apparatus in the second embodiment described above, the protrusion 31 and the hole 32 are taken as examples for varying the weight of a resin part and the present disclosure is not limited thereto.

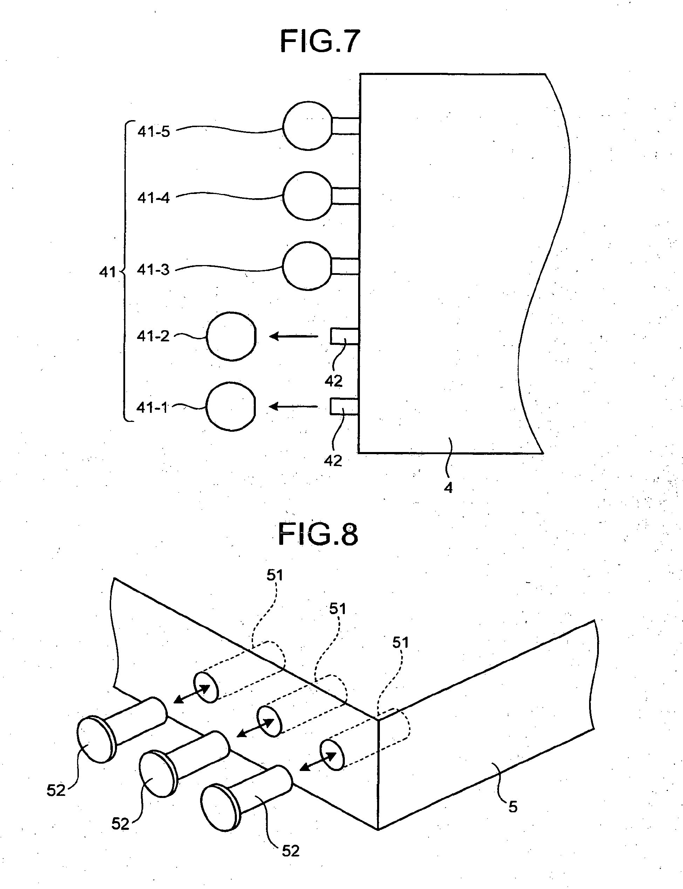

FIG. 6 is a perspective view of a principal part of a side surface of a resin part 4 according to a modification and FIG. 7 is a plane view of the resin part 4 shown in FIG. 6. As shown in FIGS. 6 and 7, the resin part 4 according to the present modification has a configuration in which a plurality of weight adjusting members 41 which allow adjusting the weight via a pin 42 are integrally formed, for example, on its side surface. For example, five weight adjusting members 41 (41-1, 41-2, 41-3, 41-4, and 41-5) each via the pin 42 are formed integrally with the resin part 4 in the example shown in FIG. 7.

The size and the shape of the weight adjusting member 41 are not specifically limited and any arbitrary size and shape may be adopted. Here, the size and the shape of respective weight adjusting members 41 may be the same or may be different from each other. In addition, the number of the weight adjusting members 41 integrally formed each via the pin 42 is not limited specifically, and a lighter weight of one weight adjusting member 41 enables a finer weight adjustment by increasing the number of the weight adjustment members 41. The weight adjusting member 41 provided via the pin 42 is formed at a position which does not cause an interference with other parts when parts are assembled.

In the present modification, resin parts 4 each having a different weight are realized by cutting off the weight adjusting member 41 from the pin 42. For example, FIG. 7 shows a state in which two weight adjusting members 41-1 and 41-2 are cut off among the five weight adjusting members 41 (41-1, 41-2, 41-3, 41-4, and 41-5). In the case of using this resin part 4, the number of weight adjusting members 41 to be cut off from the resin part 4 is determined so that the entire weight falls within the target weight based on the weight difference between the target weight and the entire weight of the capsule medical apparatus. Then, the resin part 4 from which the determined numbers of weight adjusting members 41 are cut off is used to manufacture the capsule medical apparatus. When the shape and the size of respective weight adjusting members 41 are different, it is only necessary that a weight adjusting member 41 to be cut off is determined so that the entire weight falls within the target weight and the resin part 4 from which the determined weight adjusting member 41 is cut off is used to manufacture the capsule medical apparatus.

According to the present modification, the weight of the resin part 4 can be lightened by cutting off the weight adjusting member 41 from the resin part 4. Therefore, when the entire weight of the capsule medical apparatus is heavier than the target weight, the weight adjusting member 41 is cut off by the number determined depending on the weight difference from the resin part 4 and thereby the weight of the capsule medical apparatus can be adjusted within the target range.

FIG. 8 is a perspective view of a principal part of a side surface of a resin part 5 according to another modification. As shown in FIG. 8, a plurality of holes 51 are formed on a side surface of the resin part 5 and a plurality of insertion members 52 which are detachably attached to the holes 51 are prepared in the present modification. Here, the size and the shape of respective insertion members 52 are not limited specifically and it is only necessary that the holes 51 are formed in conformity to the shape of the insertion members 52. It should be noted that a lither weight of one insertion member 52 and a larger number of holes 51 enable a finer weight adjustment.

In the present modification, resin parts 5 each having a different weight are realized by attaching or detaching the insertion member 52 to or from the hole 51 of the resin part 5. Specifically, the insertion member 52 is inserted to the hole 51 and attached to the resin part 5 when the resin part 5 is required to be heavier. On the other hand, the insertion member 52 is extracted from the hole 51 and detached from the resin part 5 when the resin part 5 is required to be lighter. In the case of using the resin part 5, the number of insertion members 52 to be attached to the resin part 5 is determined so that the entire weight falls within the target weight based on the weight difference between the target weight and the entire weight of the capsule medical apparatus. Then, the resin part 5 to which the determined numbers of insertion members 52 are attached is used to manufacture the capsule medical apparatus.

According to the present modification, the weight of the resin part 5 can be made heavier or lighter by attaching or detaching the insertion member 52 to or from the hole 51 of the resin part 5. Therefore, the weight of the capsule medical apparatus can be increased or decreased and made to fall within the target weight by using the resin part 5 to which the insertion member 52 whose number is determined depending on the weight difference is attached.

In the second embodiment described above, resin parts of plural kinds each having a different weight are prepared as a resin part used for retaining and fixing a base plate to be housed in the capsule casing. Then, a resin part having a weight corresponding to the weight difference is selectively used among resin parts each having a different weight to adjust the weight of the capsule medical apparatus. In contrast to this, a plurality of parts each having a different weight except for resin parts may be prepared. In other words, a plurality of parts, each having a different weight, of another kind may be prepared as long as a protrusion, a hole, and the like can be formed without impairing a function of the part and interfering with other parts. Besides, a plurality of parts each having a different weight may be prepared with respect to a plurality of parts of different kinds constituting the capsule medical apparatus and used in combination depending on the weight difference to adjust the weight of the capsule medical apparatus.

The variation in weight of the capsule medical apparatus to be manufactured may be suppressed by selecting a part as an assembly target depending on it weight. An example of including parts A and B as parts constituting the capsule medical apparatus is assumed. In the present modification, each weight of all parts A prepared for manufacturing the capsule medical apparatus is first measured. Then, the parts A are sorted into a plurality of groups depending on the weight. For example, an average weight of the parts A is calculated and the parts A are sorted, depending on the weight, into "a group of parts whose weight is heavier than the average weight (group A1)", "a group of parts whose weight is equivalent to the average weight (group A2)", and "a group of parts whose weight is lighter than the average weight (group A3)". Similarly, each weight of all of the prepared parts B is measured. Then, an average weight of the parts B is calculated and the parts B are sorted, depending on the weight, into "a group of parts whose weight is heavier than the average weight (group B1)", "a group of parts whose weight is equivalent to the average weight (group B2)", and "a group of parts whose weight is lighter than the average weight (group B3)".

After that, the capsule medical apparatus is supposed to be manufactured by using the parts A and B and when a part A belonging to the group A1 is used on this occasion, a part B belonging to the group B3 is used. On the other hand, when a part A belonging to the group A3 is used, a part B belonging to the group B1 is used. This allows preventing using heavy parts or light parts in combination in manufacturing one capsule medical apparatus. Therefore, the variation in weight of the capsule medical apparatus can be suppressed as a result.

While the present modification described above is configured to sort the parts A and the parts B each into groups depending on the weight, the group sorting is not necessarily performed and the parts A and the parts B may be used selectively in order of weight. Specifically, a part A which is the heaviest among the parts A and a part B which is the lightest among the parts B are selected and used in combination to manufacture the capsule medical apparatus. Next, a part A which is the second heaviest among the parts A and a part B which is the second lightest among the parts B are selected and used in combination to manufacture the capsule medical apparatus. A combination of parts A and B may be selected sequentially in order of weight in this manner and thus variation in weight of the capsule medical apparatus can be suppressed similarly to the modification described above.

To make the density of the capsule medical apparatus fall within a desired range, it is important to suppress not only the variation in weight but also the variation in volume. In case of positioning parts by causing the parts to be in direct contact with each other in assembly, the capsule medical apparatus cannot be assembled as previously arranged if any inclusion such as dirt intervenes between the directly-contacting surfaces, which can be a factor of the variation in volume of the capsule medical apparatus. As a solution, the above-described directly-contacting surfaces may be cleaned prior to the assembly of parts. This allows assembling parts after surely removing any inclusion intervening between the directly-contacting surfaces and suppressing the variation in volume. Besides, in case of using an adhesive agent in the vicinity of the directly-contacting surfaces in assembling parts, the parts are assembled after surely defining an application range of the adhesive agent to prevent the adhesive agent used from coming out onto the directly-contacting surfaces. This allows further suppressing the variation in volume.

(Third embodiment)

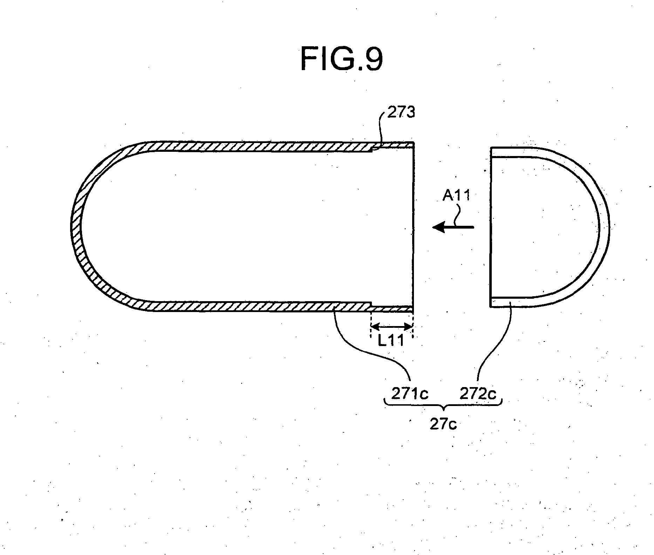

Next, a third embodiment will be explained. FIGS. 9 to 11 are explanatory views of processes in a method for manufacturing a capsule medical apparatus according to the third embodiment. The third embodiment is configured to adjust a volume which is one of the physical parameters of the capsule medical apparatus within a target range set in advance and thus to suppress the variation in density of the capsule medical apparatus. More specifically, a volume adjusting jig 6 is used and a length in the longitudinal direction of a capsule casing 27c (entire length of the capsule casing 27c) is regulated to a specified capsule length to adjust the volume. Here, a container 271c and a cap 272c are fitted to each other along the longitudinal direction of the capsule casing 27c. Therefore, the entire length of the capsule casing 27c corresponds to an end-to-end length in the fitting direction of the capsule casing 27c. The specified capsule length corresponds to the entire length of the capsule casing 27c which makes the volume of a capsule medical apparatus 2c fall within the target range, and is set in advance depending on the target volume.

FIG. 9 shows the container 271c and the cap 272c of the capsule casing 27c as an exterior member of the capsule medical apparatus. Here, the container 271c has nearly cylindrical shape whose one end has an opening and the other end has a dome-like shape and the cap 272c has a dome-like shape, similarly to what is shown in FIG. 2. In the present manufacturing method, an edge part of the cap 272c is inserted and fitted to the opening of the container 271c, after all parts constituting the capsule medical apparatus are assembled and housed in the inside of the container 271c, to seal the inside of the container 271c.

Specifically, a step part 273 is formed on an inner wall at the one end side of the container 271c according to the third embodiment so that the diameter at the one end part becomes larger than that at the inner side, and the edge part of the cap 272c is inserted and fitted to the step part 273. On this occasion, the edge part of the cap 272c slidably moves within a range L11 of a depth of the step part 273 (a width of the side wall of the step part 273). In the third embodiment, the volume of the capsule medical apparatus is adjusted within the target range by a position of an edge face of the cap 272c in the step part 273. The target range indicates a predetermined range whose median is the target value of a volume (target volume) of the capsule medical apparatus which needs adjusting. Specifically, the step part 273 serves as a volume adjuster as one example of an adjuster that adjusts the volume of the capsule medical apparatus.

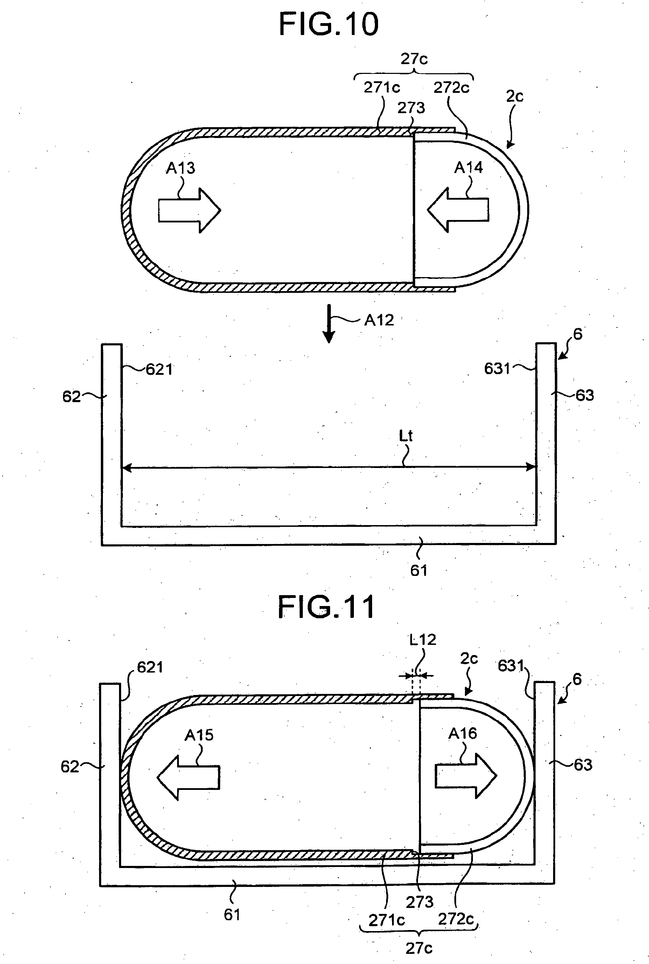

First, the edge part of the cap 272c is inserted and fitted to the opening at one end side of the container 271c (fitting process) as shown by an arrow A11 in FIG. 9. Then, the edge part of the cap 272c is directly made to slide and move along the inner wall at the one end side of the container 271c and the edge face of the cap 272c is made to directly contact the step part 273 as shown at an upper part in FIG. 10. After that, the capsule casing 27c is set to the volume adjusting jig 6 prepared in advance with the edge face of the cap 272c directly contacting the step part 273 of the container 271c as shown by an arrow A12 in FIG. 10.

Here, the capsule casing 27c is formed by a plurality of exterior members, i.e., the container 271c and the cap 272c and fixed by jointing the two members. Therefore, to make the entire length which is a length in the longitudinal direction of the capsule casing 27c (the fitting direction of the cap 272c with respect to the container 271c) constant, it is necessary to joint and fix the container 271c and the cap 272c by surely making the two members directly contact in a positional relation where the entire length of the capsule casing 27c becomes an appropriate length. In other words, when the positional relation therebetween in jointing gets out of alignment, the entire length of the capsule casing 27c varies and the volume of the capsule medical apparatus varies accordingly.

An inner pressure of the capsule casing 27c increases in fitting the container 271c and the cap 272c as described above. Specifically, air in the inside of the capsule casing 27c is compressed as shown by arrows A13 and A14 in FIG. 10 and the inner pressure increases when the edge part of the cap 272c is inserted and fitted to the opening at the one end side of the container 271c. If being left in this state, the cap 272c floats up by a force working in a direction of separating the container 271c and the cap 272c (in a direction shown by arrows A15 and A16 in FIG. 11) due to the inner pressure. Therefore, it is necessary to maintain a condition in which the container 271c and the cap 272c are kept in an appropriate positional relation in jointing the container 271c and the cap 272c.

The jig 6 serves to keep the container 271c and the cap 272c in the appropriate positional relation while using the inner pressure in the inside of the capsule casing 27c as described above. Specifically, the jig 6 regulates the entire length of the capsule casing 27c to a specified capsule length Lt. It should be noted that the specified capsule length Lt is defined as a length of the capsule casing 27c that can be adjusted as long as the edge face of the cap 272c is positioned within the range L11 (see FIG. 9) of the depth of the step part 273 of the container 271c.

Specifically, the jig 6 according to the third embodiment shown in FIG. 10 is provided with a bottom plate 61 and two supporting plates 62 and 63 which each serve as a supporter arranged perpendicularly to the bottom plate 61, and has a square-bracket shape in a side surface view. A width of the bottom plate 61 is set such that a length between side walls 621 and 631 at inner sides of the supporting plates 62 and 63 corresponds to the specified capsule length Lt. In the jig 6, the capsule casing 27c is set in such a manner that both ends of the capsule casing 27c face the side walls 621 and 631 at the inner sides of the supporting plates 62 and 63, respectively.

While the cap 272c floats up when the capsule casing 27c is set in the jig 6 by the force working due to the inner pressure as described above in the direction shown by the arrows A15 and A16 in FIG. 11, the jig 6 on this occasion regulates, by the supporting plates 62 and 63, the force which works as shown by the arrows A15 and A16 and causes the cap 272c to float, and the entire length of the capsule casing 27c to the specified capsule length Lt (regulating process). Thus, the capsule casing 27c is kept in a state where the cap 272c floats up by a length which enables the entire length of the capsule casing 27c to be the specified capsule length (by a width L12 in the example in FIG. 11). The container 271c and the cap 272c are jointed by an adhesive agent and the like in this state and fixed (jointing process). This configuration allows suppressing the variation in entire length of the capsule casing 27c due to the variety in assembly and the like and adjusting the volume of the capsule medical apparatus 2c to be manufactured within the target range. Here, a process of measuring the volume may be performed after jointing the container 271c and the cap 272c.

As explained above, the third embodiment is configured to obtain in advance the specified capsule length which is the entire length of the capsule casing 27c (length in the longitudinal direction) which enables the capsule medical apparatus 2c to have a desired volume and to prepare the jig 6 which regulates the entire length of the capsule casing 27c to the specified capsule length. Then, the capsule casing 27c which is assembled by fitting the cap 272c to the container 271c is configured to be set to the jig 6. This enables causing the cap 272c to float due to the inner pressure under an environment in which a floating amount of the cap 272c with respect to the container 271c is regulated and maintaining, between the container 271c and the cap 272c, the positional relation in which the entire length becomes the specified capsule length. Then, the container 271c and the cap 272c can be jointed in this state and fixed. Therefore, since the container 271c and the cap 272c can be surely made to contact directly with each other and fixed in the positional relation in which the entire length of the capsule casing 27c becomes the specified capsule length, the volume of each capsule medical apparatus to be manufactured can be made to fall within the desired target range and the variation in density determined depending on the volume of the capsule medical apparatus can be suppressed.

As the jig 6 shown in FIG. 10 and the like, a plurality of jigs each having a different specified capsule length Lt, i.e., each having a different length between the side walls 621 and 631 at the inner sides of the supporting plates 62 and 63 may be prepared and an appropriate jig may be selectively used depending on a target volume of a capsule medical apparatus which needs adjusting.

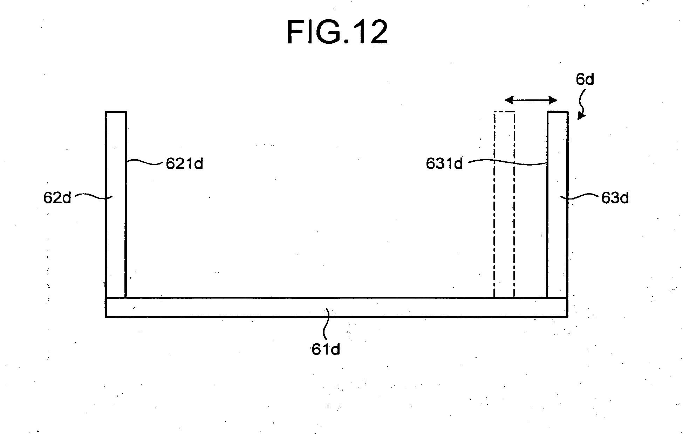

Alternatively, a jig whose specified capsule length Lt can be changed depending on the target volume may be prepared and used. FIG. 12 is a side view of a jig 6d according to a modification. The jig 6d shown in FIG. 12 is provided with a bottom plate 61d and two supporting plates 62d and 63d arranged perpendicularly to the bottom plate 61d, similarly to the jig 6 shown in FIG. 10 and the like. In the present modification, the supporting plate 63d is slidably provided to be movable with respect to the bottom plate 61d. By sliding the supporting plate 63d with respect to the bottom plate 61d, a length between side walls 621d and 631d at inner sides of the supporting plates 62d and 63d is configured to be changeable. More specifically, a width of the bottom plate 61d is set to be at least longer than a maximum length assumed as the specified capsule length and the length between the side walls 621d and 631d is configured to be changeable between the maximum length and a minimum length assumed as the specified capsule length. In jointing the container 271c and the cap 272c by using the jig 6d, it is only necessary to slide the supporting plate 63d so that the length between the side walls 621d and 631d becomes the specified capsule length depending on the desired volume of the capsule medical apparatus which needs adjusting.

(Fourth embodiment)

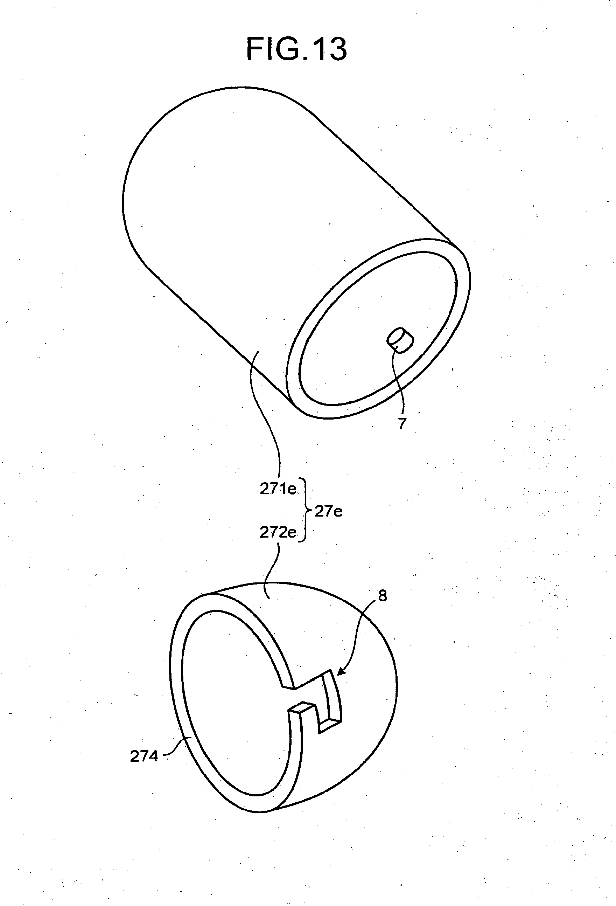

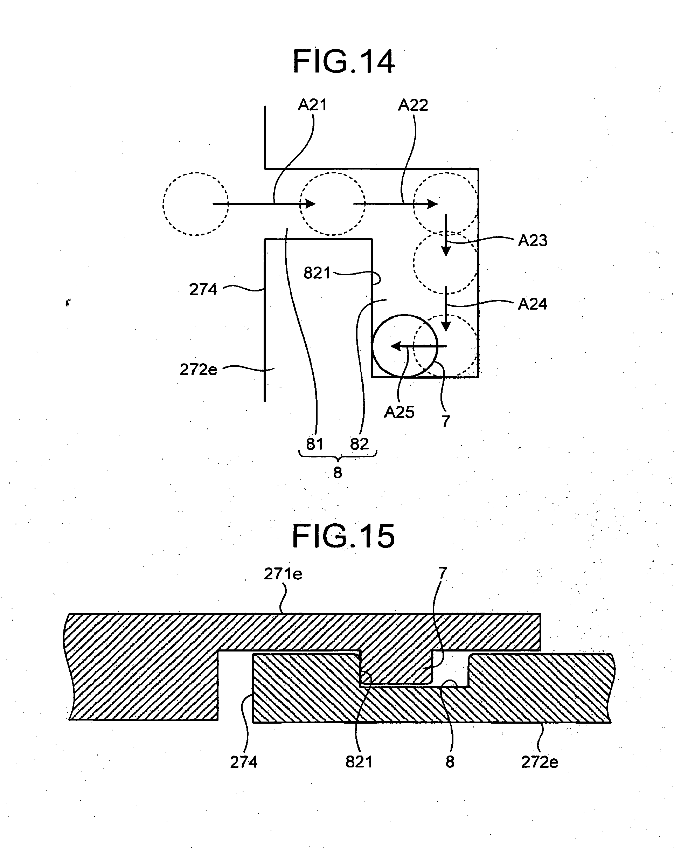

Next, a fourth embodiment will be explained. The fourth embodiment is configured to adjust the volume of the capsule medical apparatus within a target range set in advance by using an inner pressure in a capsule casing to regulate the entire length to a specified capsule length, similarly to the third embodiment. FIG. 13 is a perspective view of a container 271e and a cap 272e of a capsule casing 27e which is an exterior member of a capsule medical apparatus according to the fourth embodiment. FIG. 14 is an explanatory view of a fitting process and a regulating process of the container 271e and the cap 272e and the view showing, by arrows A21 to A25, a pathway of a protrusion part 7 which moves in a groove part 8. FIG. 15 is a cross sectional view of the fitted state of the container 271e and the cap 272e and shows a cross section, including the groove part 8, along the longitudinal direction of the capsule casing 27e.

As shown in FIG. 13, the container 271e has nearly cylindrical shape whose one end has an opening and the other end has a dome-like shape and the cap 272e has a dome-like shape, similarly to what is shown in FIG. 2 and the like. The protrusion part 7 having a cylindrical shape whose diameter is smaller than a width of a guiding groove 81, which is described later, of the cap 272e is provided on an inner wall surface at one end side where the container 271e has the opening. The groove part 8 whose one part is cut off in L shape, for example, is provided at an edge part of the cap 272e. As shown in FIG. 14, the groove part 8 includes the guiding groove 81 which has an opening on an edge face 274 of the cap 272e at one end and is orthogonal to the edge face 274, and an adjusting groove 82 which is orthogonal to the guiding groove 81 at the other end of the guiding groove 81 (i.e., parallel to the edge face 274 of the cap 272e). In the capsule casing 27e, the edge part of the cap 272e is inserted and fitted to the opening of the container 271e and the protrusion part 7 of the container 271e is slid along the groove part 8 of the cap 272e to seal the inside of the container 271e.

Here, while the protrusion part 7 and the groove part 8 are provided in pares in the container 271e and the cap 272e, respectively, the protrusion part 7 and the groove part 8 may be provided in the container 271e and the cap 272e in plural pairs. Besides, a groove having an L shape like the groove part 8 may be provided in the container 271e and a protrusion which fits in this groove like the protrusion part 7 may be provided in the cap 272e.

In fitting the cap 272e to the container 271e, the protrusion part 7 of the container 271e is first inserted and fitted to the opening of the groove part 8 of the cap 272e and slid along the guiding groove 81 (arrow A21). When the protrusion part 7 is hit against the other edge face of the guiding groove 81 (arrow A22), the protrusion part 7 is slid from one end side to the other end side of the adjusting groove 82 (arrow A23) by rotating the container 271e and the cap 272e in opposite directions, and hit against the other edge face of the adjusting groove 82 (arrow A24). Through this fitting, a force works in a direction of separating the container 271e and the cap 272e due to the inner pressure in the capsule casing 27e. As a result of this, the cap 272e floats up and the protrusion part 7 moves as far as the width of the adjusting groove 82 (arrow A25) and the protrusion part 7 comes in direct contact with a side edge face 821 at an outer side of the adjusting groove 82 (at the side of the edge face 274 of the cap 272e) as shown in FIG. 15.

Here, the protrusion part 7 of the container 271e and the groove part 8 of the cap 272e are designed so that the entire length of the capsule casing 27e in the state where the protrusion part 7 is in direct contact with the side edge face 821 of the adjusting groove 82 is equivalent to the specified capsule length, and provided in the container 271e and the cap 272e, respectively. Therefore, the volume of the capsule medical apparatus is adjusted within the target range by the protrusion part 7 provided in the container 271e and the groove part 8 provided in the cap 272e as described in the fourth embodiment. In other words, the protrusion part 7 and the groove part 8 serve as a volume adjuster as one example of an adjuster that adjusts the volume of the capsule medical apparatus.

As explained above, the positional relation which makes the entire length of the capsule casing 27e equivalent to the specified capsule length can be maintained between the container 271e and the cap 272e by fitting the protrusion part 7 of the container 271e to the groove part 8 of the cap 272e in the fourth embodiment. The container 271e and the cap 272e are jointed via an adhesive agent and the like in this state and fixed. Therefore, the container 271e and the cap 272e in the state of being surely made to directly contact with each other can be jointed and fixed in the positional relation in which the entire length of the capsule casing 27e becomes the specified capsule length. This allows suppressing the variation in entire length of the capsule casing 27e due to the variation in assembly and the like and adjusting the volume of the capsule medical apparatus to be manufactured within the target range. In addition, the length in the longitudinal direction of the capsule casing (the entire length of the capsule casing 27e) can be regulated to the specified capsule length without using the volume adjusting jig as explained in the third embodiment.

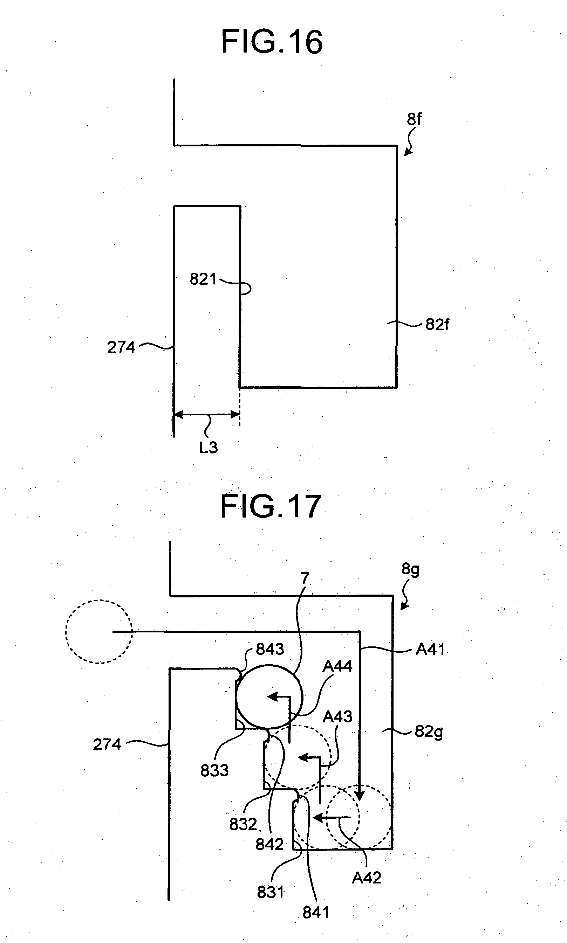

Caps of plural kinds each having a difference in position of the side edge face at the outer side of the adjusting groove (at the edge face side of the cap) may be prepared depending on the specified capsule length which enables the capsule medical apparatus to have the desired volume. Then, a cap appropriate to the target volume of the capsule medical apparatus may be selectively used. FIG. 16 shows a groove part 8f of a cap of different kind according to a modification. For example in FIG. 16, a width L3 between the edge face 274 of the cap and the side edge face 821 of an adjusting groove 82f forming the groove part 8f is smaller than what is shown in FIG. 14 and an amount of the cap float with respect to the container when the cap is fitted to the container becomes larger than that in the case of using the cap 272e in which the groove part 8 in FIG. 14 is formed. Therefore, the entire length of the capsule casing of the capsule medical apparatus to be manufactured is regulated to a length longer than that in the case of using the cap 272e in FIG. 14. Similarly, if a capsule medical apparatus in which a width between the edge face of the cap and the side edge face at the outer side of the adjusting groove forming the groove part (at the edge face side of the cap) is larger than what is shown in FIG. 14 is prepared, the entire length of the capsule casing of the capsule medical apparatus to be manufactured can be regulated to a length shorter than that in the case of using the cap 272e in FIG. 14.

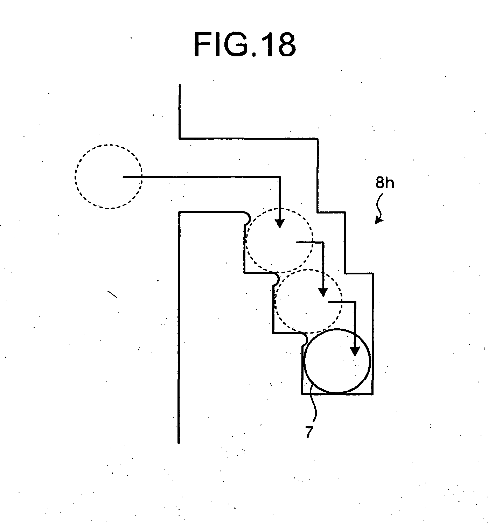

In addition, the shape of the groove part provided in the cap is not limited to the L shape. FIGS. 17 and 18 respectively show examples of groove parts 8g and 8h formed in the caps according to other modifications.

For example, the groove part 8g shown in FIG. 17 is formed such that a side edge face at an outer side of an adjusting groove 82g (at the side of the edge face 274 of the cap) has a step-like shape and provided with a step parts 831, 832, and 833. In the step parts 831, 832, and 833, locking parts 841, 842, and 843 which each lock the protrusion part 7 are formed, respectively.

In a fitting process and a regulating process according to the present modification, the protrusion part 7 provided in the container is first inserted and fitted to an opening of the groove part 8g provided in the cap and hit against the other edge face of the guiding groove 82g (arrow A41) in the same manner as the case in the fourth embodiment. Through this fitting, a force works in a direction of separating the container and the cap due to the inner pressure in the capsule casing. As a result of this, the cap floats up and the protrusion part 7 comes in direct contact with the step part 831 which is the highest step of the adjusting groove 82g (arrow A42). On this occasion, the protrusion part 7 is locked by the locking part 841 in the highest step part 831.

**EP 2 489 300 B1**

When the cap is rotated in a direction opposite to the direction in the fitting in this state, the protrusion part 7 moves to a lower step part 832 (arrow A43) and is locked by the locking part 842. When the cap is further rotated, the protrusion part 7 moves to the lowest step part 833 (arrow 44) and is locked by the locking part 843. By forming the side edge face at the outer side of the adjusting groove 82g (at the side of the edge face 274 of the cap) in a step-like shape like the present modification, the entire length of the capsule casing can be regulated to the specified capsule length by multiple steps. In this case, it is only necessary to make the protrusion part 7 located any one of the step parts 831, 832, and 833 which each enable the entire length of the capsule casing to be a corresponding specified capsule length depending on a desired volume.

On the other hand, in the case of using the cap in which the groove part 8g as shown in FIG. 18 is formed, the entire length of the capsule casing can be regulated to any one of different specified capsule lengths by multiple steps by making the protrusion part 7 move as shown by an arrow in FIG. 18 in order from the lowest step reversely compared to the case shown in FIG. 17.

While the weight adjustment of the capsule medical apparatus is explained in the first and the second embodiments, and the volume adjustment of the capsule medical apparatus is explained in the third and the fourth embodiments, these embodiments may be combined and applied. Such application enables the volume as well as the weight of the capsule medical apparatus to fall within a desired target range. Thus, each variation in weight and volume can be made small and the variation in density of the capsule medical apparatus can further be suppressed.

Besides, while the capsule medical apparatus provided with the imaging unit 21 and the illumination unit 22 in pairs is exemplified in each of the embodiments described above, the present disclosure is not limited thereto and may be applied to a so-called compound-eye capsule medical apparatus provided with the imaging unit and the illumination unit in plural pairs. For example, a binocular capsule medical apparatus includes a container which has a hollow circular cylindrical shape and openings at both ends, and an imaging unit and an illumination unit are arranged in such a manner as to be orientated to the outside of the capsule medical apparatus via a cap in each of the openings. A transparent cap is configured to be fitted to each of the openings of the container having the follow circular cylindrical shape and the binocular capsule medical apparatus is constituted by three exterior members. An application of the third embodiment, the fourth embodiment, or their modifications enables the volume to fall within a desired target range in the binocular capsule medical apparatus, too.

Industrial Applicability

As described above, the capsule medical apparatus and the method for manufacturing the capsule medial apparatus according to the present invention are suitable for suppressing a variation in the density.

Reference Signs List

| | |
|---|---|
| 1 | Weight adjusting member |
| 2, 2b | Capsule medical apparatus |
| 21 | Imaging unit |
| 22 | Illumination unit |
| 23 | Wireless communication unit |
| 24 | Permanent magnet |
| 25 | Power source unit |
| 26 | Control unit |
| 27, 27c | Capsule casing |
| 271, 271c | Container |
| 7 | Protrusion part |
| 272, 272c | Cap |
| 11 | Solid adjusting member |
| 12 | Past form adjusting member |
| 3a, 3b, 4, 5 | Resin part |
| 31 | Protrusion |
| 32 | Hole |
| 41 | Weight adjusting member |
| 51 | Hole |
| 52 | Insertion members |
| 273 | Step part |

| 274 | Edge face |
| 8, 8f, 8g, 8h | Groove part |
| 81 | Guiding groove |
| 82, 82f, 82g | Adjusting groove |
| 831, 832, 833 | Step part |
| 841, 842, 843 | Locking part |
| 6, 6d | Jig |

**Claims**

1. A method for manufacturing a capsule medical apparatus (2, 2b, 2c), comprising:

a measuring step of measuring an entire weight (We) which is a total of a weight of a capsule-shaped casing (27, 27c, 27e) and a weight of a plurality of function executing units (21-26) that are housed in the casing (27, 27c, 27e) and execute predetermined functions;
a difference calculating step of calculating a difference (Y) between the measured weight (We) and a target weight (Wt) set in advance;
a housing step of housing the plurality of function executing units (21-26) in the casing (27, 27c, 27e); and
an adjusting step of adjusting, by adding a weight adjusting member (1, 11, 12, 31, 41, 52) whose weight is equivalent to the difference (Y) between the target weight (Wt) and the entire weight (We) of all parts constituting the capsule medical apparatus (2, 2b, 2c), the weight of the casing (27, 27c, 27e) in which the plurality of function executing units (21-26) are housed.

2. The method for manufacturing a capsule medical apparatus (2, 2b, 2c) according to claim 1, wherein the adjusting step includes a selecting step of selecting a solid adjusting member (11) which has a weight equivalent to the calculated difference (Y) among a plurality of solid adjusting members (11) which are prepared in advance and each of which has a different weight, and the weight of the casing (27, 27c, 27e) in which the plurality of function executing units (21-26) are housed is increased by housing the selected solid adjusting member (11) in the casing (27, 27c, 27e) at the adjusting step.

3. The method for manufacturing a capsule medical apparatus (2, 2b, 2c) according to claim 1, wherein the adjusting step includes a determining step of determining a number of the solid adjusting members (11) which are prepared in advance and have a same weight so that a total weight of the solid adjusting members (11) becomes equivalent to the calculated difference (Y), and the weight of the casing (27, 27c, 27e) in which the plurality of function executing units (21-26) are housed is increased by housing the determined numbers of solid adjusting members (11) in the casing (27, 27c, 27e) at the adjusting step.

4. The method for manufacturing a capsule medical apparatus (2, 2b, 2c) according to claim 1, wherein the weight of the casing (27, 27c, 27e) in which the plurality of function executing units (21-26) are housed is increased, at the adjusting step, by housing a paste form adjusting member (12) which has a weight equivalent to the calculated difference (Y).

5. A method for manufacturing a capsule medical apparatus (2, 2b, 2c), comprising:

an assembling step of assembling a capsule-shaped casing (27, 27c, 27e) by arranging a plurality of function executing units (21-26) that execute predetermined functions in an inside of a plurality of exterior members (271, 272) and fitting the plurality of exterior members (271, 272) in a predetermined fitting direction with the arrangement kept;
a regulating step of regulating a length between ends in the fitting direction of the casing (27, 27c, 27e) assembled at the assembling to a length (Lt) set in advance depending on a target volume of the casing (27, 27c, 27e) in which the plurality of function executing units (21-26) are housed; and
a jointing step of jointing the exterior members (271, 272) with the length (Lt) between the ends in the fitting direction of the casing (27, 27c, 27e) regulated,
wherein a jig (6, 6d) which is provided with supporters (62, 63, 62d, 63d) that are fixed at an interval corresponding to the length (Lt) set in advance and support the ends in the fitting direction of the casing (27, 27c, 27e) is used to regulate the length between the ends in the fitting direction of the casing (27, 27c, 27e) at the regulating step.

**Patentansprüche**

1. Verfahren zum Herstellen eines medizinischen Kapselgeräts (2, 2b, 2c), das umfasst:

   einen Messschritt zum Messen eines Gesamtgewichts (We), das ein Gesamtgewicht aus einem Gewicht eines kapselförmigen Gehäuses (27, 27c, 27e) und einem Gewicht einer Mehrzahl von Funktionsausführungseinrichtungen (21-26) ist, die in dem Gehäuse (27, 27c, 27e) aufgenommen sind und vorbestimmte Funktionen ausführen;
   einen Differenzberechnungsschritt zum Berechnen einer Differenz (Y) zwischen dem gemessenen Gewicht (We) und einem im Voraus festgesetzten Zielgewicht (Wt);
   einen Aufnahmeschritt zum Aufnehmen der Mehrzahl von Funktionsausführungseinrichtungen (21-26) in dem Gehäuse (27, 27c, 27e); und
   einen Anpassungsschritt zum Anpassen des Gewichts des Gehäuses (27, 27c, 27e), in dem die Mehrzahl von Funktionsausführungseinrichtungen (21-26) aufgenommen ist, durch Hinzufügen eines Gewichtsanpasselements (1, 11, 12, 31, 41, 52), dessen Gewicht äquivalent zu der Differenz (Y) zwischen dem Zielgewicht (Wt) und dem Gesamtgewicht (We) aller Teile ist, aus denen das medizinische Kapselgeräts (2, 2b, 2c) besteht.

2. Verfahren zum Herstellen eines medizinischen Kapselgeräts (2, 2b, 2c) gemäß Anspruch 1, wobei der Anpassungsschritt einen Auswahlschritt zum Auswählen eines massiven Anpasselements (11) mit einem zu der berechneten Differenz (Y) äquivalenten Gewicht aus einer Mehrzahl von massiven Anpasselementen (11) umfasst, die im Voraus vorbereitet sind und von denen jedes ein unterschiedliches Gewicht hat, und das Gewicht des Gehäuses (27, 27c, 27e), in dem die Mehrzahl von Funktionsausführungseinrichtungen (21-26) aufgenommen ist, durch Aufnehmen des ausgewählten massiven Anpasselements (11) in dem Gehäuse (27, 27c, 27e) während des Anpassungsschritts erhöht wird.

3. Verfahren zum Herstellen eines medizinischen Kapselgeräts (2, 2b, 2c) gemäß Anspruch 1, wobei der Anpassungsschritt einen Bestimmungsschritt zum Bestimmen einer Anzahl der massiven Anpasselemente (11) umfasst, die im Voraus vorbereitet sind und ein gleiches Gewicht haben, so dass ein Gesamtgewicht der massiven Anpasselemente (11) äquivalent zu der berechneten Differenz (Y) wird und das Gewicht des Gehäuses (27, 27c, 27e), in dem die Mehrzahl von Funktionsausführungseinrichtungen (21-26) aufgenommen ist, durch Aufnehmen der bestimmten Anzahl von massiven Anpasselementen (11) in dem Gehäuse (27, 27c, 27e) während des Anpassungsschritts erhöht wird.

4. Verfahren zum Herstellen eines medizinischen Kapselgeräts (2, 2b, 2c) gemäß Anspruch 1, wobei das Gewicht des Gehäuses (27, 27c, 27e), in dem die Mehrzahl von Funktionsausführungseinrichtungen (21-26) aufgenommen ist, während des Anpassungsschritts durch Aufnehmen eines pastenförmigen Anpasselements (12) erhöht wird, das ein zu der berechneten Differenz (Y) äquivalentes Gewicht hat.

5. Verfahren zum Herstellen eines medizinischen Kapselgeräts (2, 2b, 2c), das umfasst:

   einen Montageschritt zum Montieren eines kapselförmigen Gehäuses (27, 27c, 27e) durch Anordnen einer Mehrzahl von Funktionsausführungseinrichtungen (21-26), die vorbestimmte Funktionen ausführen, im Inneren einer Mehrzahl von äußeren Elementen (271, 272) und Anpassen der Mehrzahl von äußeren Elementen (271, 272) in einer vorbestimmten Anpassrichtung, wobei die Anordnung beibehalten wird;
   einen Regulierungsschritt zum Regulieren einer Länge zwischen Enden in der Anpassrichtung des Gehäuses (27, 27c, 27e), das bei der Montage zu einer Länge (Lt) montiert wird, die im Voraus in Abhängigkeit von einem Zielvolumen des Gehäuses (27, 27c, 27e) festgesetzt wurde, in dem die Mehrzahl von Funktionsausführungseinrichtungen (21-26) aufgenommen ist; und
   einen Verbindungsschritt zum Verbinden der äußeren Elemente (271, 272), wobei die Länge (Lt) zwischen den Enden in der Anpassrichtung des Gehäuses (27, 27c, 27e) reguliert wird,
   wobei eine Vorrichtung (6, 6d), die mit Stützen (62, 63, 62d, 63d) versehen ist, welche in einem der im Voraus festgesetzten Länge (Lt) entsprechenden Abstand fixiert sind und die Enden in der Anpassrichtung des Gehäuses (27, 27c, 27e) abstützen, dazu benutzt wird, die Länge zwischen den Enden in der Anpassrichtung des Gehäuses (27, 27c, 27e) bei dem Regulierungsschritt zu regulieren.

**Revendications**

1. Procédé de fabrication d'un appareil médical de type capsule (2, 2b, 2c), comprenant :

   une étape de mesure consistant à mesurer un poids total (We) qui est un total d'un poids d'un boîtier en forme de capsule (27, 27c, 27e) et d'un poids d'une pluralité d'unités d'exécution de fonctions (21 à 26) qui sont logées dans le boîtier (27, 27c, 27e) et exécutent des fonctions prédéterminées ;
   une étape de calcul de différence consistant à calculer une différence (Y) entre le poids mesuré (We) et un poids cible (Wt) établi à l'avance ;
   une étape de logement consistant à loger la pluralité d'unités d'exécution de fonctions (21 à 26) dans le boîtier (27, 27c, 27e) ; et
   une étape d'ajustement consistant à ajuster, en ajoutant un élément d'ajustement de poids (1, 11, 12, 31, 41, 52) dont le poids est équivalent à la différence (Y) entre le poids cible (Wt) et le poids total (We) de toutes les parties constituant l'appareil médical de type capsule (2, 2b, 2c), le poids du boîtier (27, 27c, 27e) dans lequel la pluralité d'unités d'exécution de fonctions (21 à 26) sont logées.

2. Procédé de fabrication d'un appareil médical de type capsule (2, 2b, 2c) selon la revendication 1, dans lequel l'étape d'ajustement comprend une étape de sélection consistant à sélectionner un élément d'ajustement solide (11) qui présente un poids équivalent à la différence calculée (Y) parmi une pluralité d'éléments d'ajustement solides (11) qui sont préparés à l'avance et dont chacun présente un poids différent, et le poids du boîtier (27, 27c, 27e) dans lequel la pluralité d'unités d'exécution de fonctions (21 à 26) sont logées est augmenté en logeant l'élément d'ajustement solide (11) sélectionné dans le boîtier (27, 27c, 27e) à l'étape d'ajustement.

3. Procédé de fabrication d'un appareil médical de type capsule (2, 2b, 2c) selon la revendication 1, dans lequel l'étape d'ajustement comprend une étape de détermination consistant à déterminer un nombre d'éléments d'ajustement solides (11) qui sont préparés à l'avance et présentent un poids identique de sorte qu'un poids total des éléments d'ajustement solides (11) devient équivalent à la différence calculée (Y), et le poids du boîtier (27, 27c, 27e) dans lequel la pluralité d'unités d'exécution de fonctions (21 à 26) sont logées est augmenté en logeant le nombre déterminé d'éléments d'ajustement solides (11) dans le boîtier (27, 27c, 27e) à l'étape d'ajustement.

4. Procédé de fabrication d'un appareil médical de type capsule (2, 2b, 2c) selon la revendication 1, dans lequel le poids du boîtier (27, 27c, 27e) dans lequel la pluralité d'unités d'exécution de fonctions (21 à 26) sont logées est augmenté, à l'étape d'ajustement, en logeant un élément d'ajustement sous forme de pâte (12) qui présente un poids équivalent à la différence calculée (Y).

5. Procédé de fabrication d'un appareil médical de type capsule (2, 2b, 2c), comprenant :

   une étape d'assemblage consistant à assembler un boîtier (27, 27c, 27e) en forme de capsule en agençant une pluralité d'unités d'exécution de fonctions (21 à 26) qui exécutent des fonctions prédéterminées à l'intérieur d'une pluralité d'éléments extérieurs (271, 272) et en ajustant la pluralité d'éléments extérieurs (271, 272) dans une direction d'ajustement prédéterminée en conservant l'agencement ;
   une étape de régulation consistant à réguler une longueur entre les extrémités dans une direction d'ajustement du boîtier (27, 27c, 27e) assemblé à l'étape d'assemblage à une longueur (Lt) établie à l'avance en fonction d'un volume cible du boîtier (27, 27c, 27e) dans lequel la pluralité d'unités d'exécution de fonctions (26 1156) sont logées ; et
   une étape de raccordement consistant à raccorder les éléments extérieurs (271, 272) avec la longueur (Lt) entre les extrémités dans la direction d'ajustement du boîtier (27, 27c, 27e) régulée,
   dans lequel un gabarit (6, 6d), qui est prévu avec des supports (62, 63, 62d, 63d) qui sont fixés à un intervalle correspondant à la longueur (Lt) établie à l'avance et supportent les extrémités dans la direction d'ajustement du boîtier (27, 27c, 27e), est utilisé pour réguler la longueur entre les extrémités dans la direction d'ajustement du boîtier (27, 27c, 27e) à l'étape de régulation.

# FIG.1

WEIGHT ADJUSTING MEMBER ~1

WEIGHT
DIFFERENCE
Y [g]

TARGET WEIGHT
Wt [g]

ENTIRE
WEIGHT
We (g)

PART A: a [g]

PART B: b [g]

PART C: c [g]

⋮

# FIG.2

SOLID ADJUSTING
MEMBER

# FIG.3

# FIG.4

# FIG.5

32
32
32

3b

# FIG.6

42
42

41
41

4

# FIG.7

# FIG.8

# FIG.9

273

A11

L11

271c    272c

27c

# FIG.10

# FIG.11

# FIG.12

6d

621d

631d

62d

63d

61d

# FIG.13

## FIG.14

A21　A22

274

821

272e

A23

A24

A25　7

81　82

8

## FIG.15

271e　7

274　821　8　272e

## FIG.16

## FIG.17

# FIG.18

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006263167 A

- US 20070106112 A1